## Europäisches Patentamt

(19) **European Patent Office**

**Office européen des brevets**

(11) Publication number: **0 130 950**
**B1**

(12) **EUROPEAN PATENT SPECIFICATION**

(45) Date of publication of patent specification: **11.04.90**

(51) Int. Cl.⁵: **A 61 N 5/06**

(21) Application number: **84830202.2**

(22) Date of filing: **03.07.84**

(54) Radiating electromechanical apparatus for treating scalp against baldness.

(30) Priority: **04.07.83 IT 2191983**

(43) Date of publication of application:
**09.01.85 Bulletin 85/02**

(45) Publication of the grant of the patent:
**11.04.90 Bulletin 90/15**

(84) Designated Contracting States:
**AT BE CH DE FR GB LI NL SE**

(56) References cited:
**DE-A-2 740 969**
**DE-A-2 823 615**
**DE-A-3 203 922**
**FR-A-1 495 668**

(73) Proprietor: **Tessiore, Adriano**
**Via San Damiano, 5/7**
**I-16035 Rapallo (Genova) (IT)**

(72) Inventor: **Tessiore, Adriano**
**Via San Damiano, 5/7**
**I-16035 Rapallo (Genova) (IT)**

(74) Representative: **Cicogna, Franco**
**Ufficio Internazionale Brevetti Dott.Prof. Franco**
**Cicogna Via Visconti di Modrone, 14/A**
**I-20122 Milano (IT)**

Courier Press, Leamington Spa, England.

## Description

The present invention relates to an electromechanical radiating apparatus for treating the scalp against baldness.

As it is known many persons are affected by a progressive baldness because of a plurality of not completely identified factors.

It is also known that there are presently available many products against baldness, which however have provided poor results.

The French document FR—A—1 495 668 discloses a radiating apparatus comprising at least one laser source, the output beam of which is conveyed through optical fibers, and including means for varying the impinging points of the individual rays to maintain a direction perpendicular to the scalp.

Accordingly, the task of the present invention is to provide a radiating electromechanical apparatus effective to benefically affect the bulbs of the patient scalp.

Within that task, it is a main object of the present invention to provide such a radiating electromechanical apparatus which is effective to progressively stop the hair loss.

Another object of the present invention is to provide a radiating electromechanical apparatus which may be used in an easy and safe way.

Further characteristics and advantages of the radiating electromechanical apparatus according to the present invention will become more apparent thereinafter from the following detailed description of a preferred embodiment whereof, being illustrated, by way of example and not of limitation, in the accompanying drawing, where:

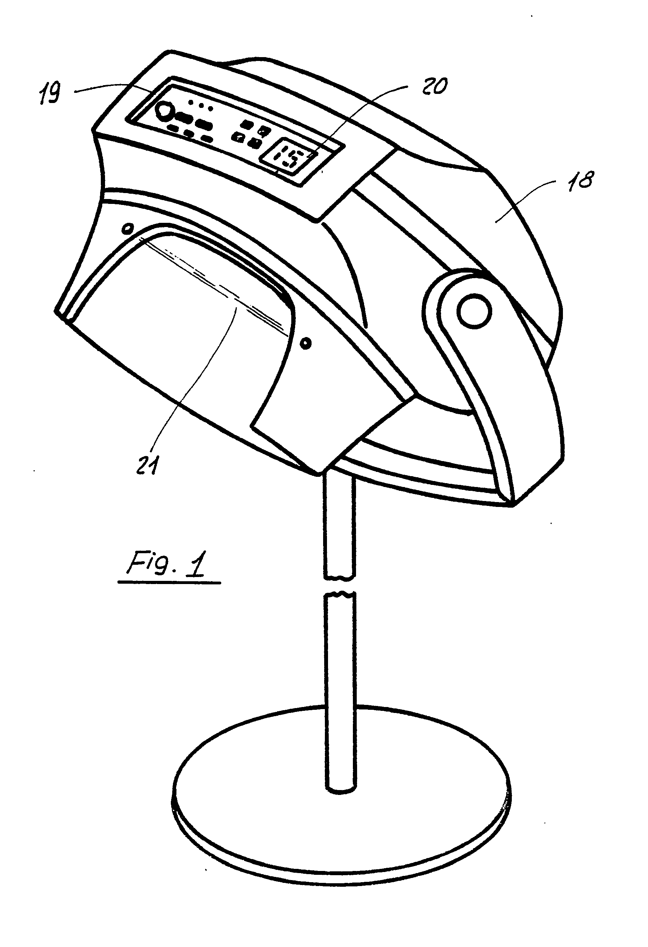

Fig. 1 is a schematic perspective view illustrating a casing of the subject apparatus;

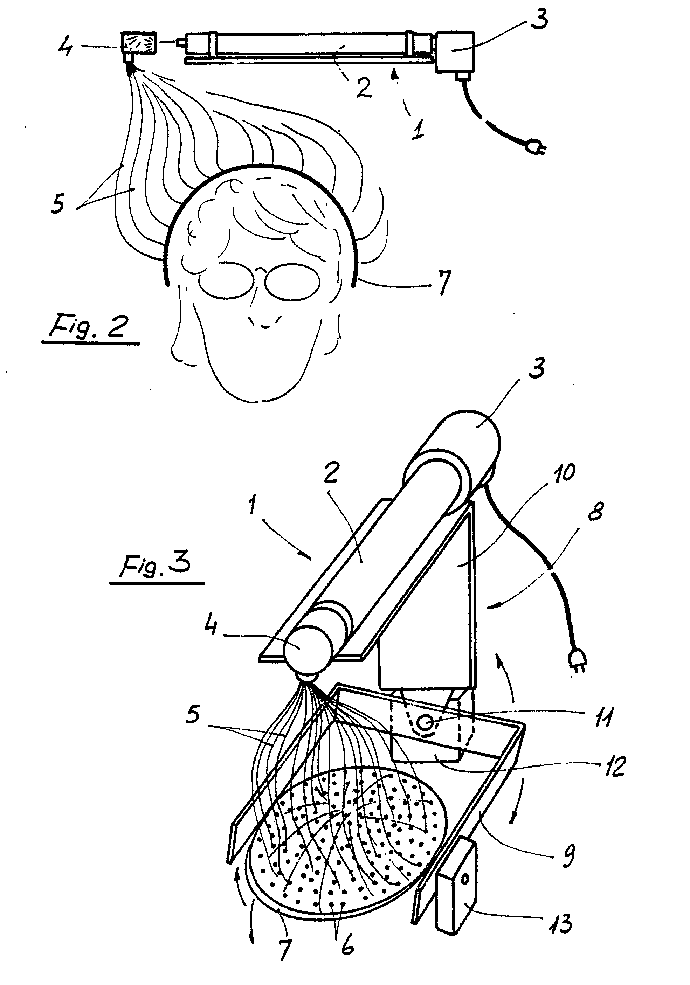

Fig. 2 illustrates a first constructional diagram of that same apparatus;

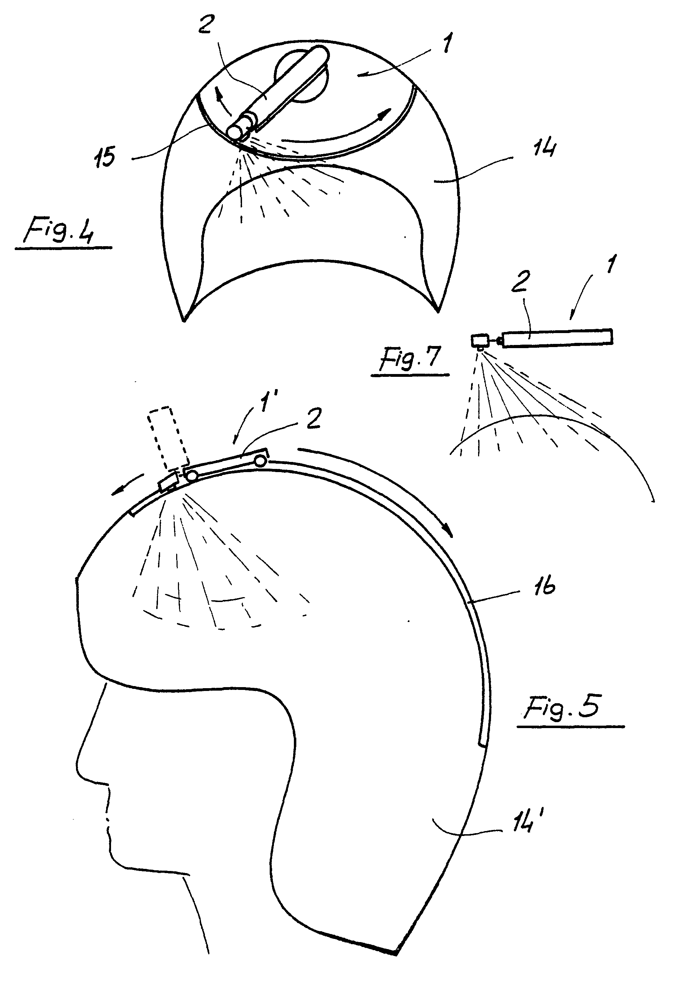

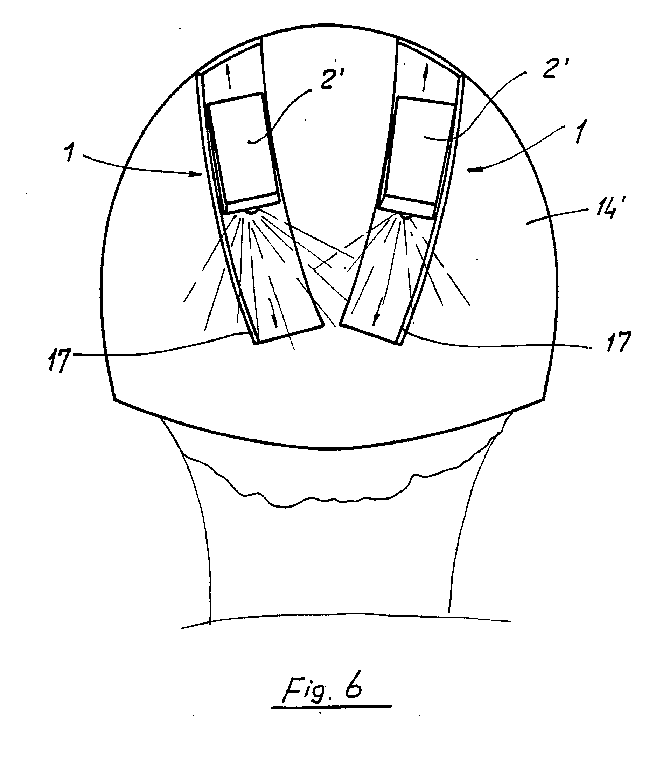

Fig. 3 is a schematic view illustrating a movable assembly or "equipment" for supporting the laser source and a perforated bowl thereon there are welded the optic fibers conveying the electromagnetic radiations produced by the laser source itself;

Figs. 4, 5 and 6 illustrate possible applications of the laser source directly on a bowl suitably designed and equipped;

Fig. 7, in particular, illustrates the preferred direction assumed by the electromagnetic radiations at the output of the offsetting-diffusing device associated with the laser source;

Fig. 8 illustrates a substantially semispherical helmet member with the optic fibers coupled through a focalizing connector;

Fig. 9 is a cross-sectional view illustrating the detail of a focalizing connector;

Fig. 10 is a cross-sectional exploded view illustrating a focalizing connector.

With reference to the figures of the accompanying drawings, the radiating electromechanical apparatus according to the present invention comprises, essentially, a laser source (1) enclosed in a suitable box-like body 2, of tubular (2) or flattened (2') shape, and energized by a suitable power supply (3).

The laser source which is preferably of the helium-neon type has its output coupled to an offsetting-diffusing device or member (4), effective to diffract the electromagnetic radiation into a plurality of beams and to convey the latter in a single beam the axis whereof has a substantially perpendicular direction with respect to the axis of said laser source.

The mentioned diffracted rays or beams impinge on a plurality of optic fibers (5) which are coupled to corresponding small holes (6), formed on the surface of a spherical bowl, 7.

The latter is mounted on a movable assembly or equipment (8), consisting of an open frame (9) articulated on a bracket element (10) supporting the laser source and effective to swing about its pivot axis (11) as actuated by a suitable motorized kinematic assembly (12).

Moreover, the mentioned spherical bowl (7) is pivoted on the frame (9), at two diametrically opposite points whereof, and is also subjected to a swinging movement about its pivot axis, substantially perpendicular to the axis (11), as actuated by a further motorized kinematic assembly (13).

The combination of the two mentioned swinging motions, in the practice, causes the scalp to be evenly impinged upon by the electromagnetic radiations, as conveyed by the optic fibers.

Alternatively, it is possible to cause the laser source to move directly on a bowl (14) or (14'), of suitable design, by rotating along a circular guide (15) or translating along guides (16) extending on substantially circumpherential lines.

In the latter case, as it is shown in fig. 6, it is possible to provide for the use of a laser source pair (1') moving along corresponding converging guides (17).

The laser source-movable equipment assembly or the laser source-bowl assembly is enclosed in a helmet member (18), of suitable shape, provided with a front portion (19) thereon there are arranged all of the controls necessary for the proper operation of the apparatus, and a display (20) is further provided controlled by a programmable timing device.

In particular, the operation of the apparatus depends, for safety reasons, on the interruption of a light beam (21) as transmitted to a photocell, mounted on the front portion of the helmet.

Owing to that approach, as soon as the user removes the helmet, the operation of the apparatus will be prevented.

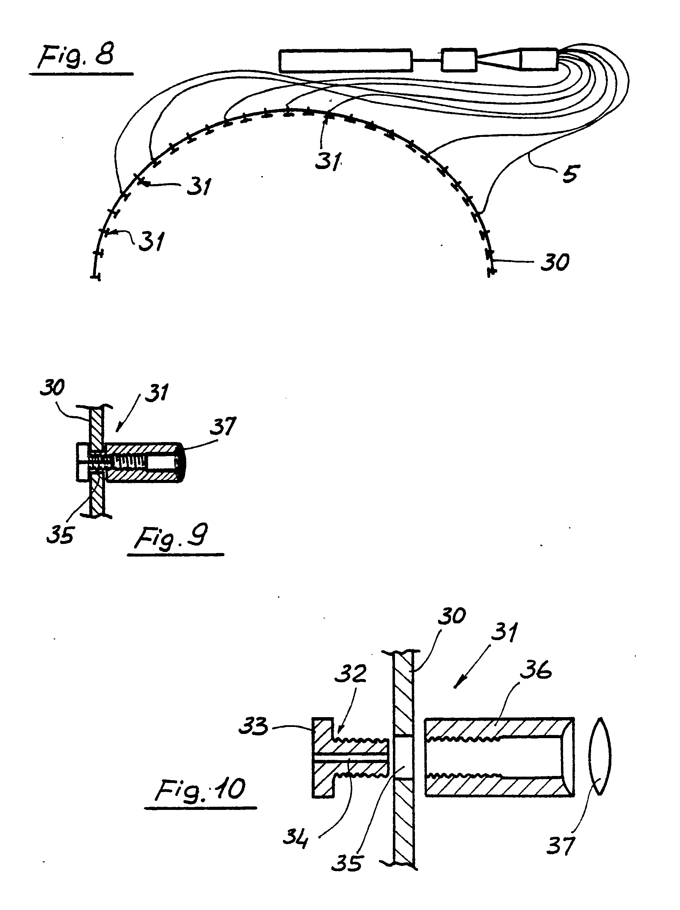

As it is illustrated in figs. 8 to 10, there is provided a small bowl helmet (30) thereon there are coupled optic fibers, indicated at (5), arranged in such a way as to simultaneously encompass all of the scalp to be treated.

In particular, the optic fibers are associated with the small helmet (30) by means of a connector (31) provided with a threaded pin (32) having a flange (33) and an axial hole (34) where the optic fiber (5) is inserted.

The pin (32) is inserted into throughgoing holes (35) formed on the small helmet (30) and is screwed in a threaded member or bush (36) affording the possibility of better concentrating the laser beam at the desired point of the scalp.

The beams conveyed within the substantially semispheric helmet (30) are able to simultaneously irradiating all of the scalp with an incidence angle of substantially 90°.

From the above disclosure and the figures of the accompanying drawings the great functionality and use facility characterizing the radiating electromechanical apparatus according to the present invention will be self-evident.

While a preferred embodiment of the radiating apparatus has been disclosed and illustrated, it should be noted that said embodiment is susceptible to several modifications and variations all of which come within the scope of the appended claims.

## Claims

1. A radiating electromechanical apparatus for treating scalp, comprising at least a laser source (1), the output beam whereof is diffused and conveyed so as to impinge on the scalp to be treated through the overall extension thereof, means being provided for continuously varying the impinging points of the individual rays or beams on said scalp, said laser source (1) being enclosed in a box-like body (2) and being energized by a supply device (3), said laser source, of the helium-neon type, having its output coupled to a beam diffusing member (4), effective to diffract the electromagnetic radiation in a plurality of beams, said diffracted beams being conveyed on said scalp through a multiplicity of optic fibers (5), characterized in that said optic fibers are engaged in corresponding small holes (6) formed on the surface of a spherical bowl (7) privotally mounted at its diametrically opposite two points on a movable assembly consisting of an open frame (9) articulated on a bracket element (10) supporting said laser source (1).

2. An apparatus according to claim 1, characterized in that said frame (9) is able of swinging about its pivot axis (11) as actuated by a motorized kinematic assembly (12), said spherical bowl being pivoted on said frame (9) at its diametrically opposite two points substantially perpendicularly with respect to said axis (11) and being also subjected to a swinging movement about its pivot axis, as actuated by a second motorized kinematic assembly (13).

3. A radiating electromechanical apparatus for treating scalp comprising at least a laser source, the output beam of which is diffused so as to impinge on the overall scalp to be treated, means being provided for continuously varying the impinging points of the individual rays or beams on said scalp, characterized in that said laser source (2) is movably supported on a bowl (14) having at least a circumferential guide (15, 16) along which said laser source is displacable.

4. An apparatus according to claim 3, characterized in that it comprises two said laser sources (2') movably supported on a bowl (14') having two converging guides each engaged by a respective laser source of said two laser sources (2', 2'') for moving therealong.

5. An apparatus according to either claim 1 or 3, characterized in that the laser source (1)/movable equipment (9) assembly, or the laser source (1)/ spherical bowl (14, 14') assembly, are enclosed in a helmet member (18) provided with a front portion (19) thereon there are arranged all of the controls necessary for operating said apparatus, a display being provided (20) which is controlled by a programmable limiting device.

6. An apparatus according to claim 1, characterized in that said optic fibres are distributed through the overall extension of said bowl and are coupled to said bowl (20) by means of a focalizing connector (31).

7. An apparatus according to claim 6, characterized in that said focalizing connector comprises a threaded pin (32), provided with a flange (33) and an axial hole (34) for coupling to said optic fiber (5), said threaded pin (32) being effective to be inserted into holes (35) as defined through said bowl and engaging with a member (36) provided, at the other end whereof, with a focalizing lens (37).

## Patentansprüche

1. Elektromechanisches Strahlungsgerät zur Behandlung der Kopfhaut, mit wenigstens einer Laserquelle (1), deren Austrittstrahl so augestrahlt und begleitet wird, die ganze zu behandelnde Kopfhaut umzutreffen, wobei Mitteln zur kontinuerlichen Anordnung der Treffpunkte der einzelnen Strahlen auf die obengenannte Kopfhaut vorgesehen sind, wobei die obengenannte Laserquelle (1) in einem Kastenkörper (2) eingeschlossen ist, und aus einer Versorgungsvorrichtung (3) in Betrieb gesetzt wird, wobei diese Laserquelle, die helium/neonartig ist, einen mit einem Ausstrahlungsglied (4) gekoppelte Austritt, der die elektromagnetische Strahlung in mehreren Strahlen ablenken kann, verseht, wobei diesen abgelenkten Strahlen durch ein Satz Sehfibern (5) auf die obengenannte Kopfhaut begleitet werden, dadurch gekennzeichnet, dass die obengenannten Sehfibern in kleinen entsprechenden Löchern (6) eingegriffen sind, wobei diese Löchern auf die Fläche einer Kugelhaube (7) geformt sind, und diese Kugelhaube in zwei ganz entgegengesetzten Stellungen auf einem verstellbaren System drehmontiert ist, wobei dies System aus einem geöffneten Rahmen (9) besteht, und dieser auf einer die obengenannte Laserquelle (1) tragenden Bügelelement (10) gelenkt ist.

2. Gerät nach Anspruch 1, dadurch gekennzeichnet, dass der obengenannte Rahmen (9), wenn aus einem kinematischen Motoreinheit (12) in Betrieb gesetzt, um seine Umdrehungsachse (11) schwingen kann, wobei die obengenannte Kugelhaube um den zwei senkrechte der obengenannten Achse (11), ganz entgegengesetzten Stellungen des obengenannten Rahmen (9) abgebolzen und um ihre Achse, wenn aus einer zweiten kinematischen Motoreinheit (13) in Betrieb gesetzt, gezwungen wird.

3. Elektromechanisches Strahlungsgerät zur Behandlung der Kopfhaut, mit wenigstens einer Laserquelle, deren Austrittstrahl so augestrahlt wird, die ganze zubehandelnde Kopfhaut umzutreffen, wobei Mitteln zur kontinuerlichen Änderung der Treffpunkte der einzelnen Strahlen auf die obengenannte Kopfhaut vorgesehen sind, dadurch gekennzeichnet, dass die obengenannte Laserquelle (2) auf einer Haube (14), die wenigstens eine Umlaufführung (15, 16) zur Verschiebung der obengenannten Laserquelle verseht, verschiebbar gelagert wird.

4. Gerät nach Anspruch 3, dadurch gekennzeichnet, dass es mit zwei Laserquellen (2') vorgesehen ist, die auf einer Haube (14') verschiebbar gelagert sind, wobei diese Haube zwei zusammenlaufende Führungen, die je aus einer entsprechenden Laserquelle der zweien Laserquellen (2', 2'') engagiert werden, zur Verschiebung deren entlang versehen.

5. Gerät nach Anspruch 1 oder 3, dadurch gekennzeichnet, dass die Laserquelle (1)/verschiebbare Ausrüstung (9)-Einheit oder die Laserquelle (1)/Kugelhaube (14, 14')-Einheit in einem helmförmigen Element (10) eingeschlossen sind, das mit einer Stirnseite (19) auf die alle notwendigen Antriebe zur Betätigung des Geräts angeordnet ist, wobei einer aus ein programmierbaren Begrenzvorrichtung angetriebener Ausrichter (Display) (20) vorgesehen ist.

6. Gerät nach Anspruch 1, dadurch gekennzeichnet, dass die obengenannten Sehfibern um die ganze Fläche der obengenannten Haube verteilt, und mit dieser Haube (20) über eine Brennstrahlkoppelung (31) gekoppelt sind.

7. Gerät nach Anspruch 6, dadurch gekennzeichnet, dass die obengenannte Brennstrahlkoppelung eine gewindete Spindel (32) verseht, die mit einem Flansch (33) und einer axialen Bohrung (34) zur Koppelung mit der obengenannten Sehfibern (5) vorgesehen ist, wobei diese gewindete Spindel (32) in den durch die Haube bestimmten Bohrungen (35) eingefügt werden kann, und durch ein auf die andere Ende vorgesehene Element (36) mit einem Brennstrahlglas (37) engagiert sich.

**Revendications**

1. Appareil électromécanique émetteur de rayons pour le traitement du cuir chevelu, comprenant au moins une source laser (1) dont le rayon de sortie est diffusé et convoyé ainsi de toucher le cuir chevelu à être traité sur toute son extension, il y étant prévu moyens pour varier en continu les points d'impact des rayons individuels sur ledit cuir chevelu, ladite source laser étant contenue dans un corps en forme de boîte (2) et étant opérée par un dispositif d'alimentation (3), ladite source laser, du genre hélium/néon, ayant sa sortie accoupliée avec un moyen (4) de diffusion du rayon qui est en état de diffracter la radiation électro-magnétique dans plusieurs rayons, lesdits rayons diffractés étant convoyés sur ledit cuir chevelu par une série de fibres optiques (5), caractérisé en ce que lesdites fibres optiques sont engagées dans petits trous (6) correspondants, formés sur la surface d'une calotte sphérique (7) montée pivotante sur deux points diamétralement opposés d'un appareillage mouvable se formant d'un cadre ouvert (9) articulé sur un élément (10) en forme de bride supportant ladite source laser (1).

2. Appareil selon la revendication 1, caractérisé en ce que ledit cadre (9) peut osciller autour de son axe (11) ou pivot s'il est opéré par une unité cinématique (12) à moteur, ladite calotte sphérique étant pivotée sur ledit cadre (9) dans ses deux points diamétralement opposés, essentiellement perpendiculaires par rapport audit axe (11), et étant aussi oscillés autour de son axe s'il est opéré par une deuxième unité cinématique (13) à moteur.

3. Appareil électromécanique émetteur de rayons pour le traitement du cuir chevelu, comprenant au moins une source laser dont le rayon de sortie est diffusé ainsi de toucher tout entier le cuir chevelu à être traité, il y étant prévu moyens pour varier en continu les points d'impact des rayons individuels sur ledit cuir chevelu, caractérisé en ce que ladite source laser (2) est supportée movible sur une calotte (14) ayant au moins une guide périphérique (15, 16) le long de laquelle ladite source laser peut être déplacée.

4. Appareil selon la revendication 3, caractérisé en ce qu'il comprend deux desdites sources laser (2') supportées movibles sur une calotte (14') pourvue de deux guides convergentes, chacune engagée par une source laser respective des deux sources laser (2', 2'') pour se déplacer le long de ladite calotte.

5. Appareil selon la revendication 1 ou 3, caractérisé en ce que l'unité formée par la source laser (1) et l'équipage movible (9), ou bien l'unité formée par la source laser (1) et la calotte sphérique (14, 14'), sont sontenues dans un élément en forme de casque (18) pourvu d'une région frontale (19) sur laquelle il y a logé toute commande nécessaire pour opérer ledit appareil, il y étant prévu un afficheur (20) qui est commandé par un dispositif limiteur programmable.

6. Appareil selon la revendication 1, caractérisé en ce que lesdites fibres optiques sont partagées sur toute l'extension de ladites calotte et sont jointes à ladites calotte (20) au moyen d'un connecteur de focalisation (31).

7. Appareil selon la revendication 6, caractérisé en ce que ledit connecteur de focalisation comprend un tourillon fileté (32) pourvu d'une bride (33) et d'un trou axial (34) pour l'accouplement avec ladite fibre optique (5), ledit tourillon fileté pouvant être introduit dans trous (35) comme définis à travers ladite calotte et s'engageant avec un élément (36) pourvu, sur l'autre extrémité de celle-ci, avec une loupe de focalisation (37).

Fig. 1

_Fig. 2_

_Fig. 3_

2

Fig.4

Fig.7

Fig.5

Fig. 6

*Fig. 8*

31

31

31

5

30

30

31

37

35

*Fig. 9*

30

31

36

32

33

34

35

37

*Fig. 10*